# EUROPÄISCHE PATENTSCHRIFT

(11) **EP 1 247 092 B1**
(45) Veröffentlichungstag und Bekanntmachung des Hinweises auf die Patenterteilung: **20.12.2006**
(21) Anmeldenummer: 01909416.8
(22) Anmeldetag: 05.01.2001
(51) Int. Cl.: G01N 33/48

(54) **MOBILES HANDGERÄT MIT WIEDERVERWENDBAREM BIOSENSOR**
MOBILE HAND-HELD UNIT COMPRISING A REUSABLE BIOSENSOR
APPAREIL PORTATIF MOBILE MUNI D'UN BIOCAPTEUR REUTILISABLE

(30) Priorität: 05.01.2000 DE 20000122 U
(43) Veröffentlichungstag der Anmeldung: 09.10.2002
(73) Patentinhaber: ABT Advanced Bioanalytical Technology GmbH, 01454 Radeberg (DE)
(72) Erfinder: KLIMES, Norbert, 13187 Berlin (DE); PFEIFFER, Dorothea, 13156 Berlin (DE); SZEPONIK, Jan, 13187 Berlin (DE); SCHELLER, Frieder, 16341 Zepernick (DE); HELD, Fred, 22299 Hamburg (DE)
(74) Vertreter: Baumbach, Friedrich
(86) Internationale Anmeldenummer: PCT/DE2001/000002
(87) Internationale Veröffentlichungsnummer: WO 2001/050126

(56) Entgegenhaltungen:
- EP-A- 0 460 343
- EP-A- 0 754 944
- WO-A-97/43640
- DE-U- 20 000 122
- DE-U- 29 511 566

## Beschreibung

Die Erfindung betrifft ein Handgerät zur Untersuchung von biologischen Flüssigkeiten wie Vollblut, Liquor, Urin und Serum ohne aufwendige Präanalytik mit Hilfe von elektrochemischen Biosensoren.

Biosensoren werden seit geraumer Zeit als empfindliche und selektive Detektionsmethoden in der Analytik, insbesondere der medizinischen Diagnostik verwendet. Dazu werden die entsprechenden Biosensoren in Analysatoren eingebracht, die in zentralen Laboratorien mit hohem Probenanfall eine schnelle, preiswerte und vor allem richtige Untersuchung der entsprechenden Parameter zulassen. Ein Probendurchsatz von 80 - 180/h ist dazu in der Regel notwendig.
Die Analysatoren basieren auf unterschiedlichen Prinzipien. Größtenteils arbeiten sie mit einem kontinuierlichen, luftsegmentierten Fluß der entsprechenden hochverdünnten Laborproben, das heißt, die Methode bedarf mit der notwendigen Verdünnung des Analysenmaterials einer Vorbereitung der zu untersuchenden Lösungen (z.B. Vollblut). Einzelproben sind auf diesen Aparaturen sehr aufwendig zu analysieren, da die Effektivität der Analysatoren erst nach der Inbetriebnahme bei Abarbeiten hoher Probenzahlen zum Tragen kommt (ESAT, EBIO, ECA).
Weiterhin sind Anordnungen bekannt, die Einzelproben, auch ohne Präanalytik, messen können, die allerdings auf Grund von hohem integrierten Meßkomfort sehr unbeweglich und damit für den dezentralen Einsatz unbrauchbar sind (YSI, NOVAstat).
Gegenwärtig kommerzialisierte mobile Systeme sind mit integrierten Biosensoren auf Grund von hohem Meßkomfort (interne Kalibrierung) zu teuer, haben zu hohen Wartungsaufwand und arbeiten mit zu großen Transportwegen für die zu messende Probe. Daraus ergeben sich neben einem relativ hohen Preis Verzögerungen und Verunreinigungen, die sich nachteilig auf die analytische Qualität auswirken.
Die im dezentralen Einsatz befindlichen Meßsysteme zur Bestimmung von Parametern wie Glucose und Lactat basieren momentan alle auf nicht wiederverwendbaren Verbrauchsmaterialien (Teststreifen, photometrisch; Streifenelektrode, amperometrisch). Damit ist die Analyse durch den Preis des Teststreifens determiniert und der Teststreifen ist nicht kalibrierbar, da nur einmal verwendbar.

Als deutliche Verbesserung ist das in GM 29511566.1 beschriebene Mobile Handgerät mit Biosensor' anzusehen. In diesem Gerät wird aufgrund eines wiederverwendbaren Biosensors eine Kalibrierung des Sensors ermöglicht und damit eine verbesserte Qualität der Analyse im Vergleich zu den Teststreifen realisiert. Weitere Vorteile liegen in einer kostengünstigeren Gestaltung der Analyse. Gegenüber den im Labor mit hoher Qulalität durchgeführten Analysen zeichnet sich das in GM 29511566.1 beschriebene Handgerät durch Mobilität bei gleichbleibender Qualität aus, d.h. mit diesem Gerät können Analysen dezentral und unabhängig von Labor mit hoher Qualität durchgeführt werden. Als nachteilig hat sich im praktischen Einsatz die technische Realisierung des Meßablaufs ergeben. Die parallele Bedienung von 2 Bedienelementen ist kompliziert und läßt Bedienfehler zu.

Aufgabe der Erfindung war es deshalb, die technische Lösung für die Realisierung eines dezentral einsetzbaren Gerätes zur Untersuchung von Blut, Urin, Liquor ohne Präanalytik und wiederverwendbarem Biosensor zu schaffen, das sich neben hoher Qualität und niedrigem Preis auch durch unkomplizierte Bedienung auszeichnet.

Die Aufgabe wird durch die Konstruktion eines Handgerätes mit linearer Bedienung gelöst.

Das Handgerät besteht aus einem austauschbaren elektrochemischen Biosensor , einem Systemlösungs-Vorratsbeutel und einem Abfallbeutel, einer Pumpe, einem Schlauch-Transportsystem, einem Funktionsrad mit Ventil- und Pumpsteuerungsfunktion, 4 Bedienelementen, einem Display und einer Steuereinheit für die Signalerfassung und den Gesamtablauf, die von einer Solarzelle und einem 9-V-Akku versorgt wird.
Das Gerät arbeitet mit einer Temperaturkompensation, so daß veränderte Empfindlichkeiten des Biosensors über Schwankungen der Umgebungstemperaturen im Bereich von 15 °C bis 35 °C auf der Basis einer Signal-Temperatur-Funktion ausgeglichen werden.

Als Vorratsbeutel für die Systemlösung sowie für den Abfall werden bevorzugt verschweißte PE-Folien verwendet, wobei das Vorratsvolumen ca. 1/3 und das Abfallvolumen ca. 2/3 des Gesamtvolumens einnimmt. Die Öffnung der Beutel erfolgt beim Einsatz in das Handgerät durch eine Kanüle. Beim Neueinsatz eines Vorratsbeutels ist die zweite Hälfte des PE-Beutels leer. In dem Maße wie der Vorrat zum Reinigen der Meßzelle verbraucht wird, wird der Abfallbeutel gefüllt. Nach vollständigem Verbrauch der Systemlösung wird der Gesamtbeutel entsorgt.

Der leicht austauschbare elektrochemische Biosensor ist eine Pt-Ag/AgCl-Elektrode, die entsprechend dem verwendeten Biomolekül verschiedene Substanzen registrieren kann. Zur Durchführung einer Messung wird das Funktionsrad aus der Ruheposition A (5) (READY) im Uhrzeigersinn in Position B (6) (SAMPLE) gebracht und damit das System in den meßbereiten Zustand versetzt. Die Zuführung der zu analysierenden Probe erfolgt durch Einstellen der Kapillare in die Probeöffnung des Handgerätes, die sich unmittelbar über dem Biosensor befindet. Anschließend wird das Funktionsrad in die Stellung C (7) (BUSY) gesetzt und damit die zu messende Probe vor den Biosensor transportiert. Nach erfolgter Messung wird im Display zur Entnahme der Kapillare und zur Reinigung des Systems aufgefordert. Die Kapillare wird durch den Nutzer entnommen, und mit dem Drehen des Funktionsrades in die Position A wird der Sensor gereinigt und der Meßwert angezeigt. Der Meßwert bleibt bis zur nächsten Messung im Display aktiv . Die Ausschrift "READY FOR USE" im Display gilt als Bestätigung der abgeschlossenen Reinigung, und das Gerät ist wieder meßbereit.
Als Pumpe wird bevorzugt eine Schlauchpumpe eingesetzt, die per Hand oder per Elektroenergieeinsatz bewegt wird.

Die 4 Bedienelemente sind für folgende Funktionen zuständig:
B1: Ein
B2: Menü
B3: Set-Taste
B4: Enter-Taste

Die Stromversorgung wird über einen internen 9 V Akkumulator realisiert, der vorzugsweise über eine Solarzelle aufgeladen wird bzw. durch ein netzbetriebenes Ladegerät.

Ein Biosensor hat in Abhängigkeit vom verwendeten Biomolekül eine befristete Lebensdauer, die im allgemeinen zwischen 10 und 30 Tagen liegt. Der Ablauf der Lebensdauer wird dem Nutzer im Display angezeigt. Nach Ablauf der Lebensdauer wird der Biosensor durch einen neuen ersetzt.
Der Austausch des Biosensors erfolgt durch Entriegelung der entsprechenden Verschlußkappe, der Entnahme des verbrauchten Sensors und dem einfachen Einlegen des neuen Sensors. Aus den im Display angezeigten Hinweisen wird der Nutzer bis zur Funktionsfähigkeit des neuen Sensors über die notwendigen Arbeitsschritte informiert.

Die Signalerfassung und der Gesamtablauf wird über eine Steuereinheit geregelt. Der Gesamtablauf beinhaltet den Beginn der Signalerfassung (Start), das Ende der Signalerfassung, die Ablage und Speicherung der Meßwerte, die Eichung des Gerätes und die Display-Informationen für den Bediener bezüglich der Arbeitsschritte.

Die Erfindung ermöglicht ein Minimum von 500 Messungen, ohne daß der Vorratsbeutel ausgewechselt werden muß. Ein besonderer Vorteil liegt darin, daß das Gerät unabhängig vom Einsatzort und der Einsatzzeit im Temperaturbereich von 15°C bis 35°C einsatzfähig ist.

Durch die folgenden Ausführungsbeispiele und Zeichnungen wird die Erfindung näher erläutert.
Fig.1 - Gesamtansicht des Handgerätes
Fig.2 - Prinzipdarstellung des Gerätes und der Anordnung von Vorrats- und Abfallbeutel

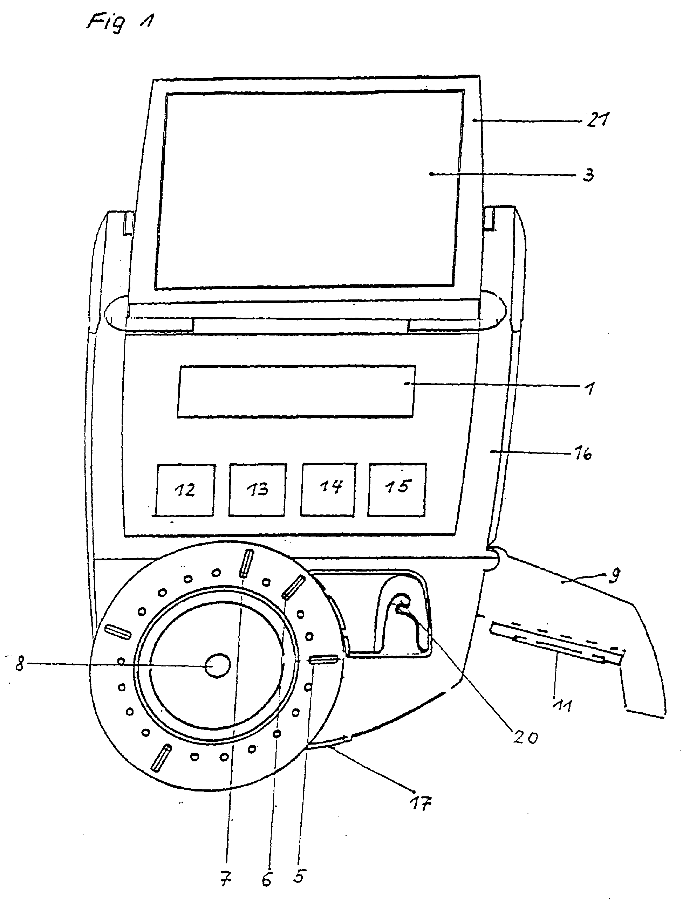

Das Handgerät gemäß Fig. 1 dient zur Untersuchung von Vollblut, Liquor, Gewebsflüssigkeit, Urin, Serum, Plasma, Lebensmittelproben sowie Wasserproben bezüglich von Metaboliten wie z.B. Glucose, Lactat; aber auch von Nährstoffen sowie Produkten der Lebensmittel- und Fermentationsindustrie wie z.B. Lactose, Ascorbinsäure, Äpfelsäure und verschiedene Aminosäuren.

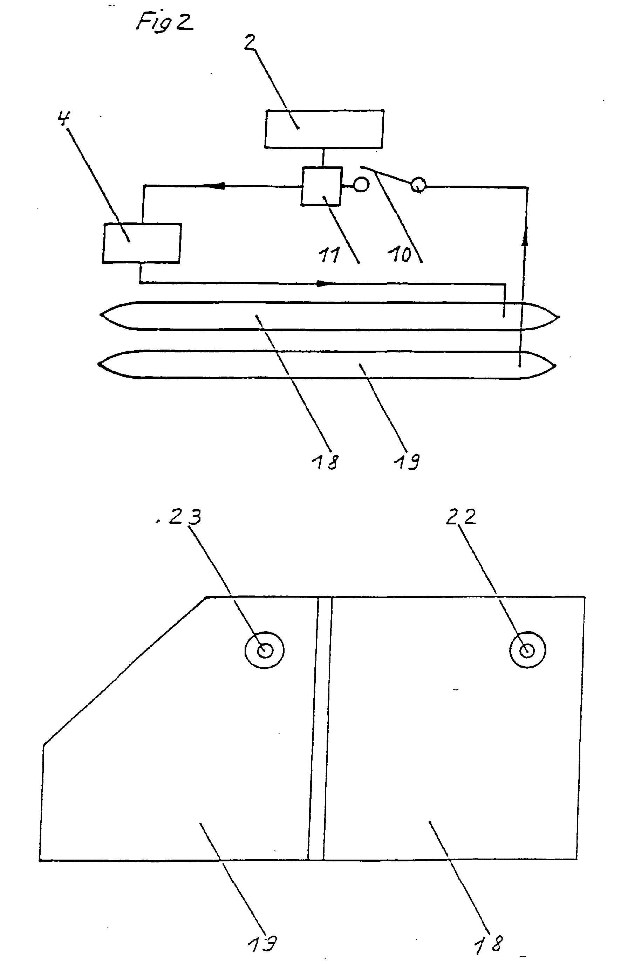

In Abbildung 1 und 2 ist der prinzipielle Aufbau eines solchen Gerätes skizziert. Der Ablauf einer Messung ist im folgenden beschrieben. Die Grund- oder Ruhestellung (stand-by) des Gerätes ist die Stellung A (5) (READY). Nach Einschalten des Gerätes über B1 (12) wird das Funktionsrad (8) in Stellung B (6) gebracht, der Nutzer über Display (1) zum Einstellen der Probe aufgefordert, diese in einer Kapillare in die dafür vorgesehene Öffnung (20) eingestellt und das Funktionsrad (8) in Position C (7) gebracht.
Nach abgeschlossener Messung wird im Display (1) der Bediener zur Entnahme der Kapillare und zum Reinigen des Systems aufgefordert. Dazu wird die Probekapillare aus der Öffnung (20) entnommen und das Funktionsrad (8) wieder in Position A (5) gedreht. Dabei wird I) der Meßwert am Display (1) angezeigt und II) Systemlösung aus dem Vorratsbeutel (19) über das Schlauchsystem durch die Sensor beinhaltende Meßzelle in den Abfallbeutel (18) bewegt. Der Kontakt des Biosensors mit der Reinigungslösung führt zur Reinigung des Sensors. Der Bediener wird mit der Ausschrift "ready for use" im Display (1) über den Abschluß der Reinigung informiert. Damit ist das Handgerät wieder meßbereit und die darauffolgende Messung läuft nach dem gleichen Schema ab.

Nach Ablauf der Lebensdauer eines Biosensors (11) (was im Display dem Bediener angezeigt wird) wird dieser ausgetauscht. Dazu wird der Bedienhebel (9) geöffnet und der alte Sensor ausgetauscht. Danach wird der Hebel (9) und damit auch das Fließsystem wieder geschlossen. Der Bediener erhält über das Display eine Führung bei der Konditionierung des Biosensors.

### Bezugszeichen:

- 1: Display
- 2: Auswerteeinheit
- 3: Solarzelle
- 4: Pumpe
- 5: Position A des Funktionsrades
- 6: Position B des Funktionsrades
- 7: Position C des Funktionsrades
- 8: Funktionsrad
- 9: Bedienhebel
- 10: Ventil
- 11: Biosensor
- 12: Bedienelement B1
- 13: Bedienelement B2
- 14: Bedienelement B3
- 15: Bedienelement B4
- 16: Gehäuse
- 17: Feder für Bedienhebel
- 18: Abfallbeutel
- 19: Vorratsbeutel
- 20: Probeöffnung
- 21: Abdeckklappe
- 22: Verschluß Abfallbeutel
- 23: Verschluß Vorratsbeutel

## Patentansprüche

1. Mobiles Handgerät mit wiederverwendbarem Biosensor, insbesondere für die dezentrale Bestimmung von originalen biologischen Lösungen, beinhaltend einen amperometrischen Biosensor (11),
eine Meßzelle,
Vorrats- (19) und Abfallbeutel (18) für frische und verbrauchte Systemlösung, eine Pumpe (4),
ein Schlauch-Transportsystem,
eine Proben-Öffnung (20) und einen Probekanal,
ein Funktionsrad (8) mit Ventil- und Pumpsteuerungsfunktion,
einen Bedienhebel für den Sensortausch (9),
ein Display (1),
4 Bedienungselemente (12, 13, 14, 15),
einen 9V-Akkumulator,
eine Solarzelle (3),
eine Auswerteeinheit für die Signalerfassung und den Gesamtablauf des Meßprozesses (2) und
ein Gehäuse (16).

2. Mobiles Handgerät nach Anspruch 1, **dadurch gekennzeichnet, daß** sich der Biosensor (11) unmittelbar unter der Probeöffnung (20) befindet.

3. Mobiles Handgerät nach Anspruch 1 und 2, **dadurch gekennzeichnet, daß** die eingesetzte Pumpe (4) per Hand oder per Elektroenergieeinsatz bewegt wird.

4. Mobiles Handgerät nach Anspruch 1-3, **dadurch gekennzeichnet, daß** für den Vorrats- (19) und den dazugehörigen Abfallbeutel (18) verschweißte PE-Folie verwendet wird, die beiden Beutel miteinander verschweißt sind, übereinander angeordnet werden und nach Verbrauch des Vorrats an Reinigungslösung gemeinsam entsorgt werden.

5. Mobiles Handgerät nach Anspruch 1-4, **dadurch gekennzeichnet, daß** die Stromversorgung der Elektrode über einen Akkumulator realisiert wird, der durch eine Solarzelle (3) oder ein netzbetriebenes Ladegerät aufgeladen wird.

6. Mobiles Handgerät nach Anspruch 1-5, **dadurch gekennzeichnet, daß** das Gewicht des Gerätes unter 500 g ist.

## Claims

1. Mobile hand-held equipment with reuseable biosensor, in particular for decentralised determination of original biological solutions, marked by
an amperometric biosensor (11),
a measuring cell,
supply (19) and waste bag (18) for fresh and used system solution,
a pump (4),
a hose transport system,
a sample opening (20) and a sample channel,
an functional wheel (8) with valve and pump control function,
a sealing lever for sensor exchange (9),
a display (1),
four operating elements (12, 13, 14, 15),
a 9V accumulator,
a solar cell (3),
an control unit for signal processing and the overall measuring process (2) and
a housing (16).

2. Mobile hand-held equipment according to Claim 1, wherein the biosensor (11) is positioned directly below the sample opening (20).

3. Mobile hand-held equipment according to Claim 1 und 2, wherein the pump (4) used is moved by hand or by the use of electrical energy.

4. Mobile hand-held equipment according to Claim 1-3, wherein welded PE foil is used for the supply (19) and the respective waste bags (18), the two bags are welded with each other, arranged one above the other and disposed jointly after consumption of the buffer supply.

5. Mobile hand-held equipment according to Claim 1-4, wherein the power supply of the electrode can be implemented via an accumulator charged via a solar cell (3) or a main supply unit.

6. Mobile hand-held equipment according to Claim 1-5, wherein the weight of the equipment is less than 500 g.

## Revendications

1. Un appareil manuel mobile avec biosensor réutilisable, en particulier pour la détermination décentralisée de solutions biologiques originales, **caractérisées par** une biosensor ampèremètre,
une cellule détectrice,
un sachet de réserve (19) et de déchets (18) pour la solution système fraîche et usée,
une pompe (4),
un système de transport à tuyau,
une ouverture à échantillon (20) et un canal à échantillon,
rad de fonction (8) avec une fonction de vous de pompage et de soupape
un levier de commande pour l'échange de sonde (9),
une visualisation (1)
4 éléments des commandes (12, 13, 14, 15),
un accumulateur 9V,
une cellule solaire (3),
une unité d'évaluation pour la saisie du signal et le déroulement complet du processus de mesure (2) et
un boîtier (16).

2. Appareil manuel mobile selon la revendication 1, **se caractérisant par le fait que** le biosensor (11) se trouve directement sous l'ouverture à échantillon (20).

3. Appareil manuel mobile selon la revendication 1 et 2, **se caractérisant par le fait que** la pompe employée (4) est déplacée à la main ou utiliser d'énergie électrique.

4. Appareil manuel mobile selon la revendication 1-3, **se caractérisant par le fait qu'**un film PE soudé est utilisé pour le sachet de réserve (19) et le sachet de déchets (18) correspondants, les deux sachets étant soudés l'un à l'autre, placés l'un au-dessus de l'autre et étant éliminés ensemble après épuisement de la réserve en tampon.

5. Appareil manuel mobile selon la revendication 1-4, **se caractérisant par le fait que** l'alimentation en courant de l'électrode pourra d'un accumulateur chargeable (3), que par d'une cellule solaire (3) ou un chargeur relié au réseau électrique est chargé.

6. Appareil manuel mobile selon la revendication 1-5, **se caractérisant par le fait que** le poids de cet appareil est inférieur à 500 g.
